(19) Europäisches Patentamt
European Patent Office
Office européen des brevets

(11) EP 2 106 470 B1

(12) **FASCICULE DE BREVET EUROPEEN**

(45) Date de publication et mention de la délivrance du brevet:
**14.09.2011 Bulletin 2011/37**

(21) Numéro de dépôt: **07870284.2**

(22) Date de dépôt: **15.11.2007**

(51) Int Cl.:
***D06B 1/00*** (2006.01) ***G09F 3/02*** (2006.01)
***D06M 23/12*** (2006.01) ***D06B 23/26*** (2006.01)
***D06P 1/00*** (2006.01) ***A61K 8/11*** (2006.01)
***A61K 9/51*** (2006.01)

(86) Numéro de dépôt international:
**PCT/FR2007/001877**

(87) Numéro de publication internationale:
**WO 2008/068417 (12.06.2008 Gazette 2008/24)**

(54) **PROCÉDÉ DE CONTRÔLE ET/OU DE SUIVI DU RELARGAGE D'AU MOINS UN PRINCIPE ACTIF IMPRÉGNÉ DANS UN TEXTILE**

STEUERUNGS- UND ODER ÜBERWACHUNGSVERFAHREN FÜR DIE ENTSALZUNG MINDESTENS EINES IN EINEN TEXTILSTOFF IMPRÄGNIERTEN WIRKSTOFFS

CONTROL AND/OR MONITORING METHOD FOR THE SALTING-OUT OF AT LEAT ONE ACTIVE INGREDIENT IMPREGNATED IN A TEXTILE<0}

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **15.11.2006 FR 0609999**

(43) Date de publication de la demande:
**07.10.2009 Bulletin 2009/41**

(73) Titulaire: **Skin'up**
**37140 Restigne (FR)**

(72) Inventeur: **BEAUGE DUGUET, Sophie**
**37140 Restigne (FR)**

(74) Mandataire: **Maureau, Philippe et al**
**Cabinet Germain & Maureau**
**12, rue Boileau**
**B.P. 6153**
**69466 Lyon Cedex 06 (FR)**

(56) Documents cités:
**EP-A- 1 353 002**
**WO-A-86/03613**
**WO-A-03/029811**
**WO-A-2006/015718**
**FR-A- 2 783 959**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 106 470 B1

Printed by Jouve, 75001 PARIS (FR)

## Description

La présente invention a trait au contrôle de l'épuisement et/ou du relargage de principes actifs qui sont imprégnés dans des textiles.
Depuis les années 1990, de multiples applications de textiles imprégnés permettant le relargage ou l'administration de principes actifs ont été développées.
A titre illustratif, on peut notamment citer les foulards imprégnés de parfum, les "lingettes" imprégnées pour l'hygiène et/ou l'entretien, les textiles d'ameublement (tels que les rideaux et/ou le linge de lit) qui relarguent des insecticides et/ou des acaricides.
Des applications combinant l'habillement et la cosmétique sont venues s'ajouter à cette liste d'applications sus énumérée. Ainsi, on a créé des habits constitués de fibres capables de relarguer des principes actifs tels que des amincissants ou des hydratants, des huiles essentielles, des vitamines, voire même des produits désodorisants.
Par ailleurs, de telles applications sont aussi connues en pharmacie, avec les pansements comportant des agents cicatrisants ou antimicrobiens.
Les applications de ces textiles imprégnés de principes actifs sont donc très variées. Mais elles ont toutes la particularité de confronter l'utilisateur de ces textiles à un même problème, qui est de ne pouvoir contrôler facilement si le textile est encore suffisamment imprégné de principes actifs, ou si au contraire, une réimprégnation du textile par lesdits principes actifs s'avère nécessaire du fait de leur relargage bien amorcé.
En effet, en fonction des conditions d'usage et du nombre de lavages du textile imprégné de principes actifs, la vitesse de relargage desdits principes actifs peut varier. A titre d'exemple, on peut citer le cas des tissus d'ameublement, dont les principes actifs dont ils sont imprégnés sont relargués de multiples façons:

- du fait de l'exposition aléatoire de ces tissus à l'air ou au soleil (conditions d'utilisation),
- du fait des traitements tels que le lavage auxquels sont soumis ces tissus.

Ainsi, de ce fait, il est difficile d'indiquer, à l'utilisateur, une « durée de vie » précise de son textile imprégné, puisqu'elle est fortement influencée par les paramètres susmentionnés.
Dans le domaine des textiles imprégnés, on connaît certes une méthode permettant de caractériser la quantité de principe actifs, et ce en fonction du nombre de lavages et des conditions de lavage. Elle consiste à analyser chimiquement, après une extraction, la quantité de principe actifs restante après chaque lavage sur le textile imprégné. Un pourcentage des principes actifs restant est donc calculé par rapport à la quantité initiale imprégnée dans le textile (voir par example FR-A-2783959).
Ainsi, avec cette méthode, l'utilisateur du textile imprégné peut être informé d'un étalonnage indiquant la quantité de principes actifs restante en fonction du nombre de lavages qu'il aura effectués, et ainsi juger de la nécessité d'effectuer une réimprégnation du textile, du fait du relargage déjà bien amorcé des principes actifs.
Cette méthode comporte toutefois les inconvénients suivants :

- elle ne prend en compte que le relargage des principes actifs par les lavages, et ne tient pas compte des conditions réelles d'utilisations qui peuvent être très diverses, selon l'utilisateur, du textile imprégné, au cours desquelles des principes actifs peuvent être aussi relargués,
- des conditions de lavages du textile imprégné employées par l'utilisateur, qui ne sont pas forcément identiques à celles qui ont été mises en oeuvre pour réaliser l'étalonnage, enfin
- elle impose à l'utilisateur du textile imprégné d'avoir à tenir les comptes du nombre de lavages réalisés.

La présente invention permet de surmonter les inconvénients sus énumérés. Elle propose en effet un procédé de contrôle, à la portée de l'utilisateur, du relargage de principes actifs imprégnés dans un textile.
La présente invention concerne un procédé de contrôle et/ou de suivi du relargage d'au moins un principe actif imprégné dans un premier textile caractérisé en ce que :

a) on dispose dudit premier textile imprégné dudit au moins un principe actif,
b) on dispose d'une étiquette de contrôle fixée sur ledit premier textile, et réalisée en un second textile au moins partiellement imprégné d'un colorant, dont la quantité est ajustée de manière à ce que la décoloration de ladite étiquette soit proportionnelle au relargage dudit au moins un principe actif,
c) on évalue la quantité du au moins un principe actif relargué par la visualisation de la décoloration de ladite étiquette.

De cette manière, l'utilisateur peut suivre l'épuisement du textile en principe actif par un simple contrôle visuel de la décoloration de l'étiquette de contrôle, et de ce fait décider :

- de la nécessité de procéder au rechargement par pulvérisation dudit textile,
- d'apposer une nouvelle étiquette, qui peut être fournie avec le contenant de l'émulsion de recharge, ou bien qui est réalisée directement par l'utilisateur, et ce par pulvérisation du colorant sur les fibres de textile constituant une nouvelle étiquette de contrôle.

Le procédé selon l'invention peut donc comprendre une étape supplémentaire consistant, après avoir observé une décoloration partielle ou complète de ladite étiquette de contrôle, à pulvériser sur ledit premier textile ledit au moins un principe actif. On peut aussi alors apposer sur ledit premier textile une nouvelle étiquette de contrôle.

Cette méthode de contrôle présente l'avantage d'éviter des rechargements intempestifs qui peuvent se révéler dangereux et aboutir à des surdosages du au moins un principe actif. Aussi, cette méthode tient compte des conditions d'utilisations telles que l'exposition à un environnement ainsi que des conditions de lavage.

Le premier textile et l'étiquette de contrôle utilisés dans le procédé selon l'invention sont des textiles identiques ou différents, qui peuvent être choisis parmi les textiles non tissés, tissés ou tricotés. Ces textiles sont constitués de fibres qui peuvent naturelles ou synthétiques, voire même être un mélange de celles-ci.

Les fibres naturelles sont choisies parmi le coton, la laine, la soie et/ou le jute.

Les fibres synthétiques sont choisies parmi les polyamides, les polyesters, les polyacryl nitrites, les polyoléfines comme le polypropylène ou le polyéthylène ou le Téflon.

Les fibres peuvent être filées, cardées ou torsadées.

L'étiquette de contrôle est fixée au premier textile. Elle peut par exemple être cousue sur une partie dudit premier textile.

Comme dit précédemment, la quantité de colorant est ajustée de manière à ce que la décoloration de ladite étiquette soit proportionnelle au relargage dudit au moins un principe actif.

Pour ce faire, un ajustage précis de la quantité de colorant imprégné dans l'étiquette de contrôle est primordial. Cet ajustage dépendra aussi de la nature des fibres de textile de l'étiquette de contrôle qui pourront influencer la vitesse de relargage du colorant.

La quantité de colorant sera donc adaptée en tenant compte des lavages, et des conditions d'utilisation du textile.

Ainsi, on pourra, pour réaliser cet ajustage, par exemple, faire des tests de suivi du relargage du au moins un principe actif en parallèle avec celui du colorant dont est imprégnée l'étiquette de contrôle. Ces tests de suivi du relargage auront pour but de simuler au mieux les conditions d'utilisation du textile imprégné d'au moins un principe actif. Par exemples, ces tests pourront simuler les conditions de port d'un vêtement, ou bien simuler les conditions environnementales auxquelles peuvent être soumis des tissus d'ameublement ou des rideaux.

En effet, le textile, auquel est destiné le contrôle et/ou le suivi du relargage d'au moins un principe actif peut être un vêtement, des gants, des chaussants, des sous-vêtements, des bas, des collants, mais aussi des rideaux, des housses de coussins ou de canapés, des textiles muraux, ou encore des articles médicaux comme les bandages, les attelles, les pansements, mais également des compresses, des bandeaux, des masques.

Le au moins un principe actif peut être choisi parmi les principes actifs pharmaceutiques, cosmétiques, les agents désodorisants, les insecticides, ou les acaricides.

Dans la présente invention, le au moins un principe actif peut être choisi parmi les principes actifs amincissants, rafraîchissants ou hydratants.

Le au moins un principe actif peut être lipophile ou hydrophile.

On citera parmi les principes actifs lipophiles :

- les vitamines liposolubles ainsi que leurs dérivés, telle la famille des rétinoïdes, par exemples rétinol, rétinaldéhyde, acide rétinoïque, des caroténoïdes, le tocophérol et ses dérivés,
- les polyphénols tels les flavonoïdes, par exemples les isoflavonoïdes, quercetine, les stylbènes, par exemples resvératrol, les catéchines, par exemples epicatechine-3-gallate, epigallocatechine-3-gallate
- les composants de parfumerie comme la vanilline, l'indole, plus généralement les huiles essentielles telles les huiles essentielles d'agrumes, de lavande
- les principes actifs pharmaceutiques liposolubles tels : fluvastatin, ketoprofen, verapamil, atenolol, griseofulvin, ranitidine.

Les principes actifs hydrophiles peuvent être choisis parmi les aminoglucosides (gentamicine), les antibiotiques (β-lactam, sulbenicilline, céfotiame, cefmenoxime), les hormones peptidiques (TRH, leuprolide, insuline), les agents anti-allergiques, antimycotiques, cytostatiques, les anxiolytiques, les contraceptifs, les sédatifs, les sels minéraux (calcium, chlore, magnésium, phosphore, potassium, sodium, soufre), les oligo-éléments (aluminium, brome, cuivre, cobalt, fer, fluor, manganèse, molybdène, iode, sélénium, silicium, vanadium, zinc), les acides aminés (alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine, valine), les peptides, les protéines, les vitamines

hydrosolubles, les polyols voire des arômes.

Dans un mode particulier de l'invention, lorsque l'étiquette de contrôle est destinée à suivre l'épuisement d'un principe actif imprégné dans un habit, le colorant dont est imprégné l'étiquette de contrôle peut être choisi dans la liste des colorants que peuvent contenir les produits cosmétiques établie à l'annexe IV de la Directive 76/768/CEE. Ainsi, si l'étiquette de contrôle est en contact avec la peau, il n'y aura pas de risque de toxicité.

Le au moins un principe actif peut être sous la forme de nanoparticules, de microparticules, ou encore en solution.

Le colorant peut être sous la forme de nanoparticules, de microparticules, ou encore en solution.

Le au moins un principe actif et le colorant peuvent sous des formes identiques ou différentes.

Dans un mode de réalisation de l'invention, le au moins un principe actif et/ou le colorant peuvent être sous la forme les microparticules qui peuvent être choisies parmi les microcapsules, les microémulsions.

Dans un mode de réalisation de l'invention, le au moins un principe actif et/ou le colorant peuvent être sous la forme de nanoparticules dont la taille est inférieure à 300 nm (0,3 $\mu$m), en moyenne de 150 nm.

Parmi les nanoparticules qui peuvent être utilisées dans la présente invention, on peut notamment citer le dioxyde de titane, l'oxyde de zinc, les fullerènes, les nanocristaux (aussi dénommés « quantum dots » ; à savoir des cristaux de semi-conducteur), ainsi que les nanoémulsions, les nanocapsules, les nanosphères, les nanovésicules ou les sphérulites.

Concernant les nanoparticules sous la forme de nanoémulsion, elles peuvent être obtenues par tous les procédés connus de l'homme de l'art permettant de réaliser des émulsions. A cet égard, on peut se référencer aux ouvrages de galénique tels que « Pharmacie Galénique », A. Le Hir, Editions Masson ($8^{iène}$ édition de 2006).

De plus, les nanoparticules peuvent être également obtenues par les procédés décrits dans les demandes de brevet EP 0717 989 A1, WO 2000/71676 A1, WO 2001/64328 A1, ainsi que W02004/060358 A2.

La demande de brevet W02004/060358 A2 décrit en effet un procédé de préparation d'émulsion PIT.

Le textile et l'étiquette de contrôle sont imprégnés respectivement du au moins un principe actif et du colorant par toute technique connue dans le domaine des textiles, telle que par exemples par trempage, bain d'épuisement, pulvérisation, foulardage.

Lorsque le au moins un principe actif est sous la forme de nanoparticules, la quantité de nanoparticules peut être comprise entre 10 et 50 g par kg de textile.

Dans un mode de réalisation préféré de l'invention, l'imprégnation est effectuée par trempage à une température comprise entre 20°C et 60°C.

En ce qui concerne le textile, outre le composé d'intérêt et/ou principe actif, la solution aqueuse dans laquelle est effectué le trempage du textile peut contenir d'autres additifs tels que ceux choisis parmi les conservateurs et/ou les antibactériens, les charges, les agents antimousses, les agents antistatiques, les stabilisants, les antioxydants et/ou les filtres UV. La solution aqueuse peut aussi comprendre des agents anti-feu, des plastifiants, des pigments, ainsi que des agents qui permettent la formation d'une gaine protectrice autour des fibres dont est constitué le textile, et qui se désagrège peu à peu au contact du sujet portant le textile.

L'invention concerne aussi une étiquette de contrôle et/ou de suivi du relargage d'au moins un principe actif imprégné dans un premier textile, ladite étiquette étant réalisée en un second textile qui est imprégné d'un colorant dont la quantité est ajustée de manière à ce que la décoloration de ladite étiquette soit proportionnelle au relargage dudit au moins un principe actif.

L'invention concerne aussi un article textile comportant une étiquette de contrôle telle que décrite ci-dessus.

L'article textile peut être choisi parmi les vêtements, les gants, les chaussants, les sous-vêtements, les bas, les collants, les rideaux, les housses de coussins ou de canapés, les textiles muraux, les bandages, les attelles, les pansements, les compresses, les bandeaux, les masques.

Les exemples suivants illustrent l'invention sans toutefois en limiter sa portée.

<u>**Exemples :**</u>

<u>La partie expérimentale se compose de trois parties illustrant la présente invention :</u>

1) La réalisation d'une étiquette de contrôle, ainsi que le suivi du relargage du colorant dont est imprégné ladite étiquette de contrôle, et ce en fonction des lavages effectués, ainsi qu'en fonction de la dilution de la solution de colorant effectuée avant l'imprégnation de l'étiquette de contrôle.

2) La mise au point d'une méthode d'extraction d'un principe actif amincissant : le stérol, pour pouvoir déterminer de manière précise la quantité restante de ce principe actif imprégné dans le textile après de multiples lavages effectués.

3) La détermination de l'étiquette de contrôle adéquate qui soit proportionnelle au relargage d'un parfum micro-encapsulé et imprégnant un textile.

Exemple 1 :

1) Réalisation d'une étiquette de contrôle :

Le protocole d'imprégnation pour la réalisation d'une étiquette de contrôle a été le suivant.
L'opération a été effectuée sur des étiquettes de contrôle de fibres de textile blanches (en 100 % polyamide non tissé).
Nature du colorant : un solide blanc, qui dissous donne une solution rose.
De numéro CAS : 13473-26-2.
Et de structure chimique :

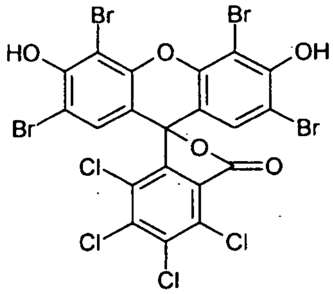

Le colorant était sous la forme d'une nanoémulsion obtenue selon le procédé décrit dans W02004/060358 A2.
La quantité de colorant présente dans la nanoémulsion était de 0,25 g pour 100 g de nanoémulsion.
La nanoémulsion a été diluée dans de l'eau selon une concentration donnée : ½ ou ¼ ou 1/8 ou 1/16.
Les étiquettes de contrôle ont été plongées à 40°C dans une des solutions diluées susmentionnées.
La durée d'imprégnation était de 30 minutes. Le séchage a été effectué à plat.
Une fois sèches, les différentes étiquettes ont été lavées jusqu'à 20 fois, et ce selon le protocole standardisé de la norme ISO 6330 (norme relative aux méthodes de lavage et de séchage domestiques en vue des essais sur des textiles).
Après chaque lavage et séchage, la couleur de l'étiquette a été numérisée.
Les résultats obtenus sont récapitulés dans le tableau 1 suivant.
Nous avons défini les couleurs suivantes :

- Rose fushia,
- Rose fushia 2 (Rose plus clair que le Rose fushia)
- Rose,
- Rose clair,
- Rose clair 2 (Rose plus clair que le Rose clair),
- Rose pastel,
- Rose pastel 2 (Rose plus clair que le Rose pastel),
- Blanc rosé.

Tableau 1 - Décoloration des étiquettes de contrôle en fonction de la dilution de la solution de coloration et du nombre de lavages effectués.

| Nbre lavages | Dilution 1/2 | Dilution 1/4 | Dilution 1/8 | Dilution1/16 |
|---|---|---|---|---|
| Témoin | Rose fushia | Rose fushia2 | Rose | Rose clair |
| Lavage 1 | Rose | Rose | Rose clair | Rose clair |
| Lavages 3 | Rose clair | Rose clair | Rose clair | Rose clair |
| Lavages 5 | Rose clair 2 | Rose clair2 | Rose clair | Rose clair |

(suite)

| Nbre lavages | Dilution 1/2 | Dilution 1/4 | Dilution 1/8 | Dilution1/16 |
|---|---|---|---|---|
| Lavages 7 | Rose pastel | Rose pastel | Rose clair | Rose pastel |
| Lavages 10 | Rose pastel2 | Rose pastel2 | Rose pastel | Rose pastel |
| Lavages 15 | Rose pastel2 | Rose pastel2 | Blanc rosé | Blanc rosé |
| Lavages 20 | Rose pastel2 | Rose pastel2 | Blanc rosé | Blanc rosé |

Exemple 2 :

2) Mise au point d'une méthode d'extraction et d'analyse d'un principe actif amincissant : le stérol.

Le stérol était sous la forme d'une nanoémulsion obtenue selon le procédé décrit dans W02004/060358 A2.

Le dosage du stérol a été réalisé par chromatographie liquide en phase inverse avec détection dans l'ultraviolet à 240 nm.

La linéarité du dosage du stérol en solution pure a été vérifiée avec des solutions de stérol préparées à partir de la source du stérol, à savoir le rhodysterol (extrait d'algue rouge connu pour ses excellentes propriétés lipolytiques).

Les réactifs étaient les suivants :

- rhodysterol (Lot 6.06.171),
- une brume amincissante SYO1 92V - Placebo (de la société COSNESSENS),
- une brume amincissante SY0192V - contenant 5 % de principe actif (de la société COSNESSENS),
- une brume amincissante SYO192X - contenant 2 % de principe actif (de la société COSNESSENS),
- du méthanol pour chromatographie (de la société VWRI),
- de l'éthanol absolu pour analyse (Carlo Erba)

Les conditions chromatographiques étaient les suivantes :

- une phase stationnaire : colonne Supelcosil LC18, 5 pm, 250x 4,6 mm,
- une phase mobile : méthanol,
- une détection UV: 240 nm,
- un débit : 1,0 mL/min,

La quantité injectée était de 20 μL.

La durée d'analyse était de 30 minutes.

Les temps de rétention (en fonction des conditions particulières) étaient de 12,5 minutes

Préparation des solutions :

Une solution mère de stérol pour le dosage (ci-après abrégée : SM) a été préparée de la manière suivante :

Dans une fiole jaugée de 50 mL, a été introduite, avec précision, une prise d'essai $P_T$ d'environ 500 mg de brume contenant du stérol à 0,075 %, qui a été dissoute dans environ 40 mL d'éthanol, et complétée au volume avec le même solvant.

Une solution témoin de dosage a été préparée en transférant 2,0 mL de la solution mère (SM) dans une fiole jaugée de 10 mL, et complétée au trait de jauge avec l'éthanol absolu.

Une solution à examiner a été préparée en introduisant dans le fond d'une fiole de 1000 mL et de façon bien tassée la moitié d'un corsaire (de poids $P_E$ en grammes), ainsi qu'un barreau magnétique.

500 mL exactement mesurés d'éthanol absolu ont été ajoutés dans la fiole qui a ensuite été portée aux ultrasons pendant 30 minutes, puis agitée magnétiquement pendant 20 minutes.

Les opérations mettant en oeuvre les ultrasons et l'agitation magnétique ont été répétées trois fois.

Une quantité exactement mesurée de 100 mL du liquide obtenu a été prélevée et évaporée à sec sous vide, à une température d'environ 40 °C.

Le résidu d'évaporation a été dissous dans 5 mL d'éthanol absolu, puis filtré sur un filtre de porosité 0,45 μm.

Deux injections de 20 μL de la solution témoin ont été réalisées afin de calculer la moyenne des aires de pic du stérol : soit $A_a$.

Deux injections de 20 μL de la solution à examiner ont été réalisées afin de calculer la moyenne des aires de

pic du stérol, soit $A_E$.

La teneur en stérol, exprimée en mg par unité de corsaire, est calculée selon la formule :

$$Q = \frac{A_E}{A_a} * \frac{2 * 0{,}075 * P_T}{50 * 5 * 100} * \frac{MM}{PE} * \frac{500 * 5}{100}$$

$A_E$ : est la moyenne des aires de pic du stérol dans la solution à examiner,
$A_a$ : est la moyenne des aires de pic du stérol dans la solution témoin,
$P_T$ : est la prise d'essai en mg de la brume à 0.075 % de stérol,
$P_E$ : est la prise d'essai en g de corsaire,
MM : est la masse en g du corsaire.

Ainsi, le dosage suivant a été effectué :

MM = 50,22212 g
$P_E$ = 24,86049 g
$P_T$ = 514 mg

Les aires suivantes de pics ont été trouvées :

$A_E$ = 48470
$A_a$ = 61928

La teneur (Q) en stérol était donc la suivante :

$$\text{La teneur Q} = \frac{48470}{61928} \times \frac{2 \times 0.075 \times 514}{50 \times 5 \times 100} \times \frac{50.22212}{24.66049} \times \frac{500 \times 5}{100} = 0.123 \text{ mg/corsaire}$$

Par ailleurs, la spécificité du dosage du stérol a été vérifiée en comparant les chromatogrammes :

- d'une solution témoin de brume exempte de stérol (1),
- d'une solution témoin de brume contenant du stérol (2),
- d'une solution témoin de rhodystérol (3),
- d'une solution échantillon obtenu après extraction (4).

On retrouvait bien dans les trois dernières solutions un pic bien isolé avec un temps de rétention de l'ordre de 12,5 minutes caractéristique de la présence de stérol.

Cela validait donc bien l'idée de réaliser l'extraction du stérol par une chromatographie liquide.

De plus, la linéarité de la méthode a été vérifiée en dosant le stérol à partir de quatre solutions préparées à partir de rhodystérol.

<u>Exemple 3 :</u>

L'exemple ci-après illustre l'épuisement par lavage de composés d'intérêts et/ou de principes actifs micro-encapsulés et fixés sur des fibres et/ou textiles de coton.

Le composé d'intérêt était un parfum : l'acétate de linalyle (principal composé de la lavande), de numéro de CAS : 115-95-7 et de structure chimique :

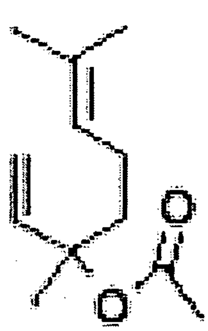

Afin que le parfum se libère des capsules fixées aux fibres, une extraction a été réalisée par extraction accélérée par solvant (ASE) avec un DIONEX. Le solvant choisi était l'acétone. L'ASE utilise un procédé à hautes pressions et hautes températures, ce qui permet de casser la membrane des capsules et d'extraire l'acétate de linalyle. Une chromatographie en phase gazeuse a alors été mise au point pour pouvoir quantifier l'acétate de linalyle extrait. Les méthodes d'extraction et d'analyse ont été réalisées sur le textile, avant et après chaque lavage.

Les résultats suivants ont été obtenus :

Tableau 2 : Pourcentage de parfum extrait du textile.

| Nbre lavages | % Parfum |
|---|---|
| Témoin | 100 |
| Lavage 1 | 70 |
| Lavages 3 | 60 |
| Lavages 5 | ≈50 |
| Lavages 7 | ≈40 |
| Lavages 10 | 35 |

Parmi les étiquettes de contrôle réalisées à l'exemple 1, on peut remarquer dans le tableau 3 suivant que l'étiquette de contrôle réalisée avec une dilution½ suit une décoloration proche de l'évolution du pourcentage de parfum extrait du textile.

Tableau 3 - Comparatif % actif/ décoloration en fonction du nombre de lavages

| Nbre lavages | Dilution 1/2 | % Parfum |
|---|---|---|
| Témoin | Rose fushia | 100 |
| Lavage 1 | Rose | 70 |
| Lavages 3 | Rose clair | 60 |
| Lavages 5 | Rose clair 2 | ≈50 |
| Lavages 7 | Rose pastel | ≈40 |
| Lavages 10 | Rose pastel2 | 35 |

Ainsi, l'étiquette de contrôle réalisée avec la dilution ½ pourrait tout à fait convenir comme étiquette de contrôle au textile imprégné de microcapsules d'acétate de linalyle.

## Revendications

1. Procédé de contrôle et/ou de suivi du relargage d'au moins un principe actif imprégné dans un premier textile

**caractérisé en ce que** :

a) on dispose dudit premier textile imprégné dudit au moins un principe actif,
b) on dispose d'une étiquette de contrôle fixée sur ledit premier textile, et réalisée en un second textile au moins partiellement imprégné d'un colorant, dont la quantité est ajustée de manière à ce que la décoloration de ladite étiquette soit proportionnelle au relargage dudit au moins un principe actif,
c) on évalue la quantité du au moins un principe actif relargué par la visualisation de la décoloration de ladite étiquette.

2. Procédé selon la revendication 1, **caractérisé en ce que** lesdits premier et second textiles sont identiques ou différents et choisis parmi les textiles non tissés, tissés ou tricotés.

3. Procédé selon l'une quelconque des revendications 1 à 2, **caractérisé en ce que** le au moins un principe actif est choisi parmi des principes actifs pharmaceutiques, cosmétiques, des agents désodorisants, des insecticides, des acaricides.

4. Procédé selon l'une quelconque des revendications 1 à 3 **caractérisé en ce que** le au moins un principe actif est choisi parmi les vitamines liposolubles ainsi que leurs dérivés, les polyphénols, les stylbènes, les catéchines, la vanilline, l'indole, les huiles essentielles, le fluvastatin, le ketoprofen, le verapamil, l'atenolol, le griseofulvin, la ranitidine, les aminoglucosides, les antibiotiques, les hormones peptidiques, les agents anti-allergiques, antimycotiques, cytostatiques, les anxiolytiques, les contraceptifs, les sédatifs, les sels minéraux, les oligo-éléments, les acides aminés, les peptides, les protéines, les vitamines hydrosolubles, les polyols, les arômes.

5. Procédé selon l'une quelconque des revendications 1 à 4 **caractérisé en ce que** ledit au moins un principe actif est sous la forme de nanoparticules, de microparticules, ou encore de solution.

6. Procédé selon l'une quelconque des revendications 1 à 5 **caractérisé en ce que** ledit colorant est sous la forme de nanoparticules, de microparticules, ou encore de solution.

7. Procédé selon l'une des revendications 5 à 6 **caractérisé en ce que** lesdites nanoparticules sont choisies parmi le dioxyde de titane, l'oxyde de zinc, les fullerènes, les nanocristaux, les nanoémulsions, les nanocapsules, les nanosphères, les nanovésicules ou les sphérulites.

8. Procédé selon l'une ou l'autre des revendications 5 et 6 **caractérisé en ce que** les microparticules sont choisies parmi les microcapsules, les microémulsions.

9. Procédé selon l'une quelconque des revendications 1 à 8 **caractérisé en ce que** l'imprégnation desdits premier et second textiles est réalisée par trempage, bain d'épuisement, pulvérisation ou foulardage.

10. Procédé selon l'une quelconque des revendications 1 à 9 **caractérisé en ce que** l'imprégnation est effectuée par trempage à une température comprise entre 20 °C et 60 °C.

11. Procédé de contrôle et/ou de suivi du relargage selon l'une quelconque des revendications 1 à 10 **caractérisé en ce que** lorsque le au moins un principe actif est sous la forme de nanoparticules, la quantité de nanoparticules est comprise entre 10 et 50 g par kg de textile.

12. Etiquette de contrôle et/ou de suivi du relargage d'au moins un principe actif imprégné dans un premier textile, **caractérisée en ce que** ladite étiquette est réalisée en un second textile qui est imprégné d'un colorant dont la quantité est ajustée de manière à ce que la décoloration de ladite étiquette soit proportionnelle au relargage dudit au moins un principe actif impregné dans ledit premier textile.

13. Article textile comportant une étiquette selon la revendication 12.

14. Procédé selon l'une quelconque des revendications 1 à 11 **caractérisé en qu'**il comprend une étape supplémentaire consistant, après avoir observé une décoloration partielle ou complète de ladite étiquette, à pulvériser sur ledit premier textile ledit au moins un principe actif.

15. Procédé selon la revendication 14, **caractérisé en ce que**, en outre, on appose sur ledit premier textile une nouvelle

étiquette de contrôle selon la revendication 12.

**Claims**

1. A method for monitoring and/or following the release of at least one active principle impregnated in a first textile, **characterized in that**:

   a) there is available said first textile impregnated with said at least one active principle,
   b) there is available a monitoring label attached to said first textile and made of a second textile at least partially impregnated with a colorant, the amount of which is adjusted so that the decolorization of said label is proportional to the release of said at least one active principle,
   c) the amount of the at least one active principle released is evaluated by observation of the decolorization of said label.

2. The method as claimed in claim 1, **characterized in that** said first and second textiles are identical or different and are chosen from nonwoven, woven or knitted textiles.

3. The method as claimed in any one of claims 1 to 2, **characterized in that** the at least one active principle is chosen from pharmaceutical or cosmetic active principles, deodorizing agents, insecticides or acaricides.

4. The method as claimed in any one of claims 1 to 3, **characterized in that** the at least one active principle is chosen from fat-soluble vitamins and their derivatives, polyphenols, stilbenes, catechins, vanillin, indole, essential oils, fluvastatin, ketoprofen, verapamil, atenolol, griseofulvin, ranitidine, aminoglucosides, antibiotics, peptide hormones, antiallergic, antimycotic or cytostatic agents, anxiolytics, contraceptives, sedatives, mineral salts, trace elements, amino acids, peptides, proteins, water-soluble vitamins, polyols or flavorings.

5. The method as claimed in any one of claims 1 to 4, **characterized in that** said at least one active principle is in the form of nanoparticles or of microparticles or else in solution.

6. The method as claimed in any one of claims 1 to 5, **characterized in that** said colorant is in the form of nanoparticles or of microparticles or else in solution.

7. The method as claimed in one of claims 5 to 6, **characterized in that** said nanoparticles are chosen from titanium dioxide, zinc oxide, fullerenes, nanocrystals, nanoemulsions, nanocapsules, nanospheres, nanovesicles or spherulites.

8. The method as claimed in either of claims 5 and 6, **characterized in that** the microparticles are chosen from microcapsules or microemulsions.

9. The method as claimed in any one of claims 1 to 8, **characterized in that** the impregnation of said first and second textiles is carried out by dipping, exhaustion bath, spraying or padding.

10. The method as claimed in any one of claims 1 to 9, **characterized in that** the impregnation is carried out by dipping at a temperature of between 20°C and 60°C.

11. The method for monitoring and/or following the release as claimed in any one of claims 1 to 10, **characterized in that**, when the at least one active principle is in the form of nanoparticles, the amount of nanoparticles is between 10 and 50 g per kg of textile.

12. A label for monitoring and/or following the release of at least one active principle impregnated in a first textile, **characterized in that** said label is made of a second textile which is impregnated with a colorant, the amount of which is adjusted so that the decolorization of said label is proportional to the release of said at least one active principle impregnated in the said first textile.

13. A textile article, comprising a label as claimed in claim 12.

14. The method as claimed in any one of claims 1 to 11, **characterized in that** it comprises an additional stage which

consists, after having observed a partial or complete decolorization of said label, in spraying said at least one active principle over said first textile.

**15.** The method as claimed in claim 14, **characterized in that**, in addition, a new monitoring label as claimed in claim 12 is affixed to said first textile.

## Patentansprüche

**1.** Kontroll- und/oder Prüfverfahren des Aussalzens mindestens eines in einen ersten Textilstoff imprägnierten aktiven Prinzips, **dadurch gekennzeichnet, dass**:

a) man über den ersten, mit dem mindestens einen aktiven Prinzip imprägnierten Textilstoff verfügt,
b) man über ein Kontrolletikett, das auf dem ersten Textilstoff befestigt wurde und aus einem zweiten Textilstoff hergestellt ist, verfügt, der zumindest teilweise mit einem Farbstoff imprägniert ist, dessen Menge derart abgestimmt ist, dass die Entfärbung des Etiketts proportional zum Aussalzen des mindestens einen aktiven Prinzips erfolgt,
c) man die Menge des mindestens einen ausgesalzenen aktiven Prinzips durch Visualisierung der Entfärbung des Etiketts ermittelt.

**2.** Verfahren nach Anspruch 1, **dadurch gekennzeichnet, dass** der erste und zweite Textilstoff identisch oder unterschiedlich sind und aus den ungewebten, gewebten oder gewirkten Textilstoffen ausgewählt sind.

**3.** Verfahren nach einem der Ansprüche 1 bis 2, **dadurch gekennzeichnet, dass** das mindestens eine aktive Prinzip aus den pharmazeutischen, kosmetischen, deodorierenden, insektiziden, akariziden aktiven Prinzipien ausgewählt ist.

**4.** Verfahren nach einem der Ansprüche 1 bis 3, **dadurch gekennzeichnet, dass** das mindestens eine aktive Prinzip aus den fettlöslichen Vitaminen sowie ihren Derivaten, den Polyphenolen, den Stilbenen, den Katechinen, dem Vanillin, dem Indol, den ätherischen Ölen, dem Fluvastatin, dem Ketoprofen, dem Verapamil, dem Atenolol, dem Griseofulvin, dem Ranitidin, den Aminoglukosiden, den Antibiotika, den Peptidhormonen, den Antiallergika, den Antimykotika, den Zytostatika, den Anxiolytika, den Kontrazeptiva, den Sedativa, den Mineralsalzen, den Oligoelementen, den Aminosäuren, den Peptiden, den Proteinen, den wasserlöslichen Vitaminen, den Polyolen, den Aromen ausgewählt ist.

**5.** Verfahren nach einem der Ansprüche 1 bis 4, **dadurch gekennzeichnet, dass** das mindestens eine aktive Prinzip in Form von Nanopartikeln, Mikropartikeln oder auch einer Lösung ist.

**6.** Verfahren nach einen der Ansprüche 1 bis 5, **dadurch gekennzeichnet, dass** der Farbstoff in Form von Nanopartikeln, Mikropartikeln oder auch einer Lösung ist.

**7.** Verfahren nach einem der Ansprüche 5 bis 6, **dadurch gekennzeichnet, dass** die Nanopartikel aus dem Titandioxid, dem Zinkoxid, den Fullerenen, den Nanokristallen, den Nanoemulsionen, den Nanokapseln, den Nanosphären, den Nanovesikeln oder den Sphäroliten ausgewählt sind.

**8.** Verfahren nach dem einem oder dem anderen der Ansprüche 5 und 6, **dadurch gekennzeichnet, dass** die Mikropartikel aus den Mikrokapseln, den Mikroemulsionen ausgewählt sind.

**9.** Verfahren nach einem der Ansprüche 1 bis 8, **dadurch gekennzeichnet, dass** die Imprägnierung des ersten und zweiten Textilstoffs durch Eintauchen, Erschöpfungsbad, Zerstäuben oder Klotzen durchgeführt wird.

**10.** Verfahren nach einem der Ansprüche 1 bis 9, **dadurch gekennzeichnet, dass** die Imprägnierung durch Eintauchen bei einer Temperatur zwischen 20°C und 60 °C inklusive durchgeführt wird.

**11.** Kontroll- und/oder Prüfverfahren des Aussalzens nach einem der Ansprüche 1 bis 10, **dadurch gekennzeichnet, dass**, wenn das mindestens eine aktive Prinzip in Form von Nanopartikeln ist, die Nanopartikelmenge zwischen 10 und 50 g inklusive pro kg Textilstoff ist.

**12.** Kontroll- und/oder Prüfetikett des Aussalzens von mindestens einem in einen ersten Textilstoff imprägnieren aktiven Prinzips, **dadurch gekennzeichnet, dass** das Etikett aus einem zweiten Textilstoff hergestellt ist, der mit einem Farbstoff imprägniert ist, dessen Menge derart abgestimmt ist, dass die Entfärbung des Etiketts proportional zum Aussalzen des mindestens einen aktiven, in den ersten Textilstoff imprägnierten Prinzips erfolgt.

**13.** Textilartikel, der ein Etikett nach Anspruch 12 aufweist.

**14.** Verfahren nach einem der Ansprüche 1 bis 11, **dadurch gekennzeichnet, dass** es einen zusätzlichen Schritt umfasst, der darin besteht, nachdem eine teilweise oder vollständige Entfärbung des Etiketts beobachtet wurde, das mindestens eine aktive Prinzip auf den ersten Textilstoff zu zerstäuben.

**15.** Verfahren nach Anspruch 14, **dadurch gekennzeichnet, dass** man weiterhin auf dem ersten Textilstoff ein neues Kontrolletikett nach Anspruch 12 anbringt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2783959 A
- EP 0717989 A1
- WO 200071676 A1
- WO 200164328 A1
- WO 2004060358 A2